# EUROPEAN PATENT SPECIFICATION

(11) **EP 1 627 924 B1**
(45) Date of publication and mention of the grant of the patent: **06.04.2011**
(21) Application number: 04090321.3
(22) Date of filing: 19.08.2004
(51) Int. Cl.: C12Q 1/68

(54) **Method for the analysis of methylated DNA**
Methode für die Analyse von methylierter DNA
Méthode d'analyse de l'ADN méthylé

(43) Date of publication of application: 22.02.2006
(73) Proprietor: Epigenomics AG, 10178 Berlin (DE)
(72) Inventor: Gütig, David, 13357 Berlin (DE); Schuster, Matthias, 13158 Berlin (DE); Distler, Jürgen, 10825 Berlin (DE)
(74) Representative: Schubert, Klemens

(56) References cited:
- US-A- 5 786 146
- US-A1- 2003 082 600
- US-A1- 2004 009 515
- LIU QIANG ET AL: "Pyrophosphorolysis-activatable oligonucleotides may facilitate detection of rare alleles, mutation scanning and analysis of chromatin structures." NUCLEIC ACIDS RESEARCH. 15 JAN 2002, vol. 30, no. 2, 15 January 2002 (2002-01-15), pages 598-604, XP002336292 ISSN: 1362-4962
- LIU QIANG ET AL: "Detection of extremely rare alleles by bidirectional pyrophosphorolysis-activated polymerization allele-specific amplification (Bi-PAP-A): measurement of mutation load in mammalian tissues." BIOTECHNIQUES. JAN 2004, vol. 36, no. 1, January 2004 (2004-01), pages 156-166, XP001207019 ISSN: 0736-6205
- FRAGA M F ET AL: "DNA METHYLATION: A PROFILE OF METHODS AND APPLICATIONS" BIOTECHNIQUES, EATON PUBLISHING, NATICK, US, vol. 33, no. 3, September 2002 (2002-09), pages 632,634,636-649, XP001107174 ISSN: 0736-6205
- HERMAN J G ET AL: "METHYLATION-SPECIFIC PCR: A NOVEL PCR ASSAY FOR METHYLATION STATUS OF CPG ISLANDS" PROCEEDINGS OF THE NATIONAL ACADEMY OF SCIENCES OF USA, NATIONAL ACADEMY OF SCIENCE. WASHINGTON, US, vol. 93, 1 September 1996 (1996-09-01), pages 9821-9826, XP002910406 ISSN: 0027-8424
- XP002296074

## Description

### Background of the invention

The present invention relates to a method for the analysis of methylated cytosines in DNA. 5-methylcytosine is the most frequent covalent base modification in the DNA of eukaryotic cells. It plays an important biological role, e.g. in the regulation of the transcription, in genetic imprinting, and in tumorigenesis (for review: Millar et al.: Five not four: History and significance of the fifth base. In: The Epigenome, S. Beck and A. Olek (eds.), Wiley-VCH Verlag Weinheim 2003, p. 3-20). Therefore, the identification of 5-methylcytosine as a component of genetic information is of considerable interest. However, a detection of 5-methylcytosine is difficult because 5-methylcytosine has the same base pairing behavior as cytosine. As a consequence, the usual methods for identifying nucleic acids are not applicable. Moreover, the epigenetic information carried by 5-methylcytosine is completely lost during PCR amplification.

The usual methods for methylation analysis operate essentially according to two different principles. In the first case, methylation-specific restriction enzymes are utilized, and in the second case, a selective chemical conversion of unmethylated cytosines to uracil is conducted (bisulfite treatment; for review: European Patent Application 103 47 400.5, filing date: Oct. 9th 2003, applicant: Epigenomics AG). In a second step the enzymatically or chemically pretreated DNA is amplified and analyzed in different ways (for review: Fraga and Esteller: DNA methylation: a profile of methods and applications. Biotechniques. 2002 Sep;33(3):632, 634, 636-49; WO 02/072880 pp. 1 ff).

A particulary important application of methylation analysis is the cancer diagnosis out of bodily fluids. Cancer cell DNA in bodily fluids has the property to be uniformly methylated over stretches of several 100 base pairs, while DNA of normal cells like blood shows a random mosaic methylation. However, a reliable diagnosis by detecting specially methylated cytosines in body fluids is difficult, because very small amounts of aberrant methylation patterns have to be found within a large amount of background DNA, which is methylated differently, but which has the same base sequence.

The methods known in the state of the art meet these requirements only to a certain extend. For sensitive detection the DNA is usually bisulfite treated and subsequently amplified in a methylation specific way by using either methylation specific primer or blocker oligonucleotides. The use of methylation specific primers is known as "MSP" (Herman et al.: Methylation-specific PCR: a novel PCR assay for methylation status of CpG islands. Proc Natl Acad Sci U S A. 1996 Sep 3;93(18):9821-6). The application of methylation specific blockers is known as "Heavy Methyl" (WO 02/072880; Cottrell et al. A real-time PCR assay for DNA-methylation using methylation-specific blockers. Nucl. Acids. Res. 2004 32: e10). Both methods can be used as a real time PCR ("MethyLight"-WO00/70090; US 6,331,393; Trinh et al.: DNA methylation analysis by MethyLight technology. Methods. 2001 Dec;25(4) :456-62).

However, the applicability of these methods for the sensitive and specific detection of methylated DNA is limited. Due to the fact that an unspecific amplification of the background DNA would cause false positive results, it is necessary to increase the specificity of the amplification by using primer or blocker sequences which contain several methylation specific positions. In return, only those sequences can be analysed that comprise several CpG position. In addition, these positions have to be comethylated.

Due to these restrictions and due to the great importance of cytosine methylation there is a special technical need for methods enabling a high performance methylation analysis. In the following such a method is described.

The present invention allows a sensitive and specific detection of cytosine methylation. Herein the chemically converted DNA is amplified using specially designed oligonucleotides. Firstly, the oligonucleotides carry a methylation specific nucleotide at the 3' end. Consequently, a mismatch free binding of the olionucleotides only takes place with the DNA of a defined methylation status. In contrast, the DNA of the of the opposite methylation status forms a mismatch with the 3' end of the oligonucleotides. Secondly, the nucleotide at the 3' end of the oligonucleotides is chemically modified in a way that the extension of the oligonucleotides is blocked. In case of binding without mismatch, however, the blocker nucleotide is removed by the exonuclease activity of the DNA polymerase. Subsequently, an extension of the oligonucleotide can occur. In contrast, the blocked 3' ends of the mismatched oligonucleotides are not digested. As a result an extension cannot take place. In this way it is possible to selectively amplify DNA of a certain methylation state while the amplification of the background DNA is inhibited. This method allows a very sensitive and specific methylation analysis.

At the same time the scope of the present invention is different than that of the already known "MSP"- and "Heavy Methyl" methods which require comethylation of several adjacent CpG position for achieving sufficient sensitivity. Here only 1 or 2 methylation specific positions in the sequences to be analysed are targeted in a very focused manner.

A similiar method for the detection of mutations is already described as "PAP" (pyrophosphorolysis-activated polymerization; e.g.: Liu and Sommer: Pyrophosphorolysis-activatable oligonucleotides may facilitate detection of rare alleles, mutation scanning and analysis of chromatin structures. Nucleic Acids Res. 2002 Jan 15;30(2):598-604 m.w.N.; Liu and Sommer: Detection of extremely rare alleles by bidirectional pyrophosphorolysis-activated polymerization allele-specific amplification (Bi-PAP-A): measurement of mutation load in mammalian tissues. Biotechniques. 2004 Jan;36(1):156-66 m.w.N.; US Patent Application 20040009515; US Patent 6,534,269).

The use of a special embodiment of the PAP technology, the litigation-mediated PAP (LM-PAP) for methylation analysis is mentioned in the US patent application 20040009515 (paragraph 0048 and 0179). However, the method described therein involves using methylation specific restriction enzymes. Therefore the applicability of this method is limited to certain sequences containing recognition sites of the restriction enzymes.

Here the combination of bisulfite conversion and PAP is described for the first time. This combination allows a sensitive and specific detection of cytosine methylation without the sequence dependency of LM-PAP. Since specific CpG positions are targeted rather than comethylated areas, the design of PAP-based methylation assays is comparably straightforward. The described method can be implemented to be highly sensitive to the methylation state of individual cytosines as opposed to the co-methylation requirement of MSP and Heavy Methyl. On the other hand, the described invention is also flexible enough to be adaptable to situations, were the detection of co-methylation is required. Due to the great importance of cytosine methylation and due to the above mentioned disadvantages in the prior art the present invention marks a significant technical progress.

### Description

The present invention provides a novel method for the analysis of cytosine methylation in DNA. The invention is characterised in that the following steps are conducted:
a) a genomic DNA sample is chemically or enzymatically treated in such a way that all of the unmethylated cytosine bases are converted to uracil while the 5-methylcytosine bases remain unchanged,
b) at least one oligonucleotide carrying a non-extendable 3' end is annealed to the converted DNA, wherein the non-extendable 3' terminus of the oligonucleotide is specific for a defined methylation status,
c) the non-extendable 3' terminus of the oligonucleotide is only removed in case the oligonucleotide is bound without mismatch to the DNA with the methylation status to be detected, while hybridization to the background DNA takes only place under mismatch formation,
d) the unblocked oligonucleotide is extended,
e) the methylation status is concluded from the absence or presence of an extended oligonucleotide product.

In the first step of the present invention a genomic DNA sample is chemically treated in such a way that all of the unmethylated cytosine bases are converted to uracil, or another base which is dissimilar to cytosine in terms of base pairing behaviour, while the 5-methylcytosine bases remain unchanged. Depending on the diagnostic or scientific question to be analysed the genomic DNA sample can be obtained from various sources, e.g. from cell lines, biopsies or tissue embedded in paraffin. According to the above mentioned advantages it is particulary preffered to analyse bodily fluids like plasma, serum, stool or urine. The genomic DNA is isolated by standard methods, as found in references such as Sambrook, Fritsch and Maniatis, Molecular Cloning: A Laboratory Manual, CSH Press, 2nd edition, 1989: Isolation of genomic DNA from mammalian cells, Protocol I, p. 9.16 - 9.19 and in the commonly used QIAamp DNA mini kit protocol by Qiagen. The conversion of unmethylated, but not methylated, cytosine bases within the DNA sample is conducted with a converting agent, preferably a bisulfite such as disulfite or hydrogen sulfite. The reaction is performed according to standard procedures (e.g.: Frommer et al.: A genomic sequencing protocol that yields a positive display of 5-methylcytosine residues in individual DNA strands. Proc Natl Acad Sci U S A. 1992 Mar 1;89(5):1827-31; Olek, A modified and improved method for bisulphite based cytosine methylation analysis. Nucleic Acids Res. 1996 Dec 15;24(24):5064-6.; DE 100 29 915; DE 100 54 317). In a preferred embodiment, the conversion is conducted in presence of a reagent that denatures the DNA duplex and of a radical scavenger (DE 100 29 915; German patent applications 10347397.1; 10347396.3; 10347400.5; 10347399.8; filing date: Oct. 9 2003, applicant: Epigenomics AG). It is also possible to conduct the conversion enzymatically, e.g by use of methylation specific cytidine deaminases (German patent application 103 31 107.6, filing date: July 4 2003, applicant: Epigenomics AG).

In the second step of the present invention at least one methylation specific oligonucleotide carrying a non-extendable 3' end is annealed to the converted DNA.

The methylation specific oligonucleotide carries a non-extendable 3' end being activatable by pyrophosphorolysis. The non-extendible 3'terminus is a nucleotide or nucleotide analog which has the capacity to form a Watson-Crick base bair with a complementary nucleotide and which lacks a 3' OH capable of being extended by a nucleic acid polymerase. In one embodiment, the non-extendible 3' terminus may be a non-extendible 3' deoxynucleotide, such as a dideoxynucleotide. In a second embodiment, the non-extendible 3' terminus may be a chemically modified nucleotide lacking the 3' hydroxyl group, such as an acyclonucleotide. Acyclonucleotides substitute a 2-hydroxyethoxymethyl group for the 2'-deoxyribofuranosyl sugar normally present in dNMPs. In other embodiments, the non-extendible 3' terminus may be other blockers as described elsewhere (US Patent Application 20040009515). Examples of a non-extendible 3' termini include, but are not limited to, a 2'3'-dideoxynucleotide, 3'-deoxyadenosine (cordycepin), 3'-azido-3'-deoxythymidine (AZT), 2',3'-dideoxyinosine (ddI),2',3'-dideoxy-3'-thiacytidine (3TC) and 2',3'-didehydro-2',3'-dide- oxythymidine (d4T).

In the third step of the present invention the non-extendable 3' terminus of the oligonucleotide is removed in case the oligonucleotide is bound without mismatch to the DNA with the methylation status to be detected. The 3' end of the oligonucleotides is activatable by pyrophosphorolysis. This means that the blocked 3' end can be removed by an enzyme that has pyrophosphorolysis activity in the presence of pyrophosphate (PP.sub.i) generating a nucleotide triphosphate. This activation specifically takes place when the 3' end of the oligonucleotide is bound to the DNA without mismatch, which means only the DNA of a certain methylation status will be activated

In a preferred embodiment DNA polymerases are used to activate the 3'termini of the oligonucleotides. Preferred DNA polymerases having pyrophosphorolysis activity are thermostable Tfl, Taq, and genetically engineered DNA polymerases, such as AmpliTaqFs and ThermoSequenase.TM.. These genetically engineered DNA polymerases have the mutation F667Y or an equivalent mutation in their active sites. The use of genetically engineered DNA polymerases, such as AmpliTaqFs and ThermoSequenase.TM., greatly improves the efficiency of PAP (Liu and Sommer 2002, loc cit). These Family I DNA polymerases can be used when the activatable oligonucleotide is a 3' dideoxynucleotide or an acyclonucleotide. When the activatable oligonucleotide is an acyclonucleotide, Family II archaeon DNA polymerases can also be used. Examples of such polymerases include, but are not limited to, Vent (exo-) and Pfu (exo-). These polymerases efficiently amplify 3' acyclonucleotide blocked Oligonucleotides. Two or more polymerases can also be used in one reaction.

The activation of the 3' end of the oligonucleotide may also occur by another mechanism, such as a 3' exonuclease.

Other enzymes usable for the activation of the oligonucleotide (e.g. helicases, topoisomerases, telomerases) are described in detail elsewhere (US patent application 20040009515).

In the fourth step of the invention the unblocked oligonucleotide is extended. The extension is preferably conducted by a nucleic acid polymerase in the presence of nucleoside triphosphates (see above, US patent application 20040009515).

In the fifth step of the present invention the methylation status is concluded form the absence or presence of an extended oligonucleotide product. In one embodiment, the detection of the nucleic acid is performed by detecting the extended oligonucleotide, e.g. by means of an incorporated labelled nucleotide. In a second aspect, the extended oligonucleotide is detected by gel electrophoresis. In a third aspect, the extended oligonucleotide is detected by the binding or incorporation of a dye or spectral material. The person skilled in the art knows further methods for the detection of the extended oligonucleotide. In a particulary preferred embodiment real time probes are used to detect the presence of the extension product. Several versions of real time probes are known, e.g. Lightcycler, Taqman, Scorpio, Sunrise, Molecular Beacon or Eclipse probes. Details concerning structure or detection of these probes are known in the state of the art (e.g. US Patent 6,331,393 with further references). For example Taqman probes can be designed by the "PrimerExpress" Software (Applied Biosystems).

In a preferred embodiment of the present invention, the PAP method described above can be performed bidirectionally (Bi-PAP). Bi-PAP uses two opposing pyrophosphorolysis activatable oligonucleotides with one nucleotide overlap at their 3' termini. Thus, in Bi-PAP, PAP is performed with a pair of opposing activatable oligonucleotides. Both the downstream and upstream oligonucleotides are specific for the nucleotide of interest at the 3' termini (e.g., an C:G base pair). In the initial round of amplification segments of undefined size are generated. In subsequent rounds, a segment equal to the combined lengths of the oligonucleotides minus one is amplified exponentially. Nonspecific amplification occurs at lower frequencies because this design eliminates misincorporation error from an unblocked upstream. The oligonucleotides may be 30-60 nucleotides for most efficient amplification (US patent application 20040009515 ).

Amplification by the PAP method can be linear or exponential. Linear amplification is obtained when the activatable oligonucleotide is the only complementary oligonucleotide used. Exponential amplification is obtained when a second opposing oligonucleotide, which may be a second activatable oligonucleotide, is present that is complementary to the desired nucleic acid strand. The activatable oligonucleotide and the second oligonucleotide flank the region that is targeted for amplification. The second oligonucleotide anneals to the separated desired nucleic acid strand product. Subsequently polymerization extends the second oligonucleotide on the desired nucleic acid strand to synthesize a copy of the nucleic acid template strand. Afterwards the synthesized nucleic acid template strand is separated from the desired nucleic acid strand. By repeating the above mentioned steps an exponential amplification can be achieved.

In a particular preferred embodiment the methylation analysis is conducted with two methylation specific oligonucleotides in combination with a methylation specific real time probe system.

The present disclosure can also profit of other modifications of PAP which are, e.g., decribed in detail in the US patent application 20040009515.

The US patent application discloses ways of performing a massively parallel analysis of DNA by using microarrays. A methylation analysis by using these high throughput methods is also part of the present invention, as far as said methods are applicable for bisulfite treated DNA.

The methods disclosed here are preferably used for the diagnosis and/or prognosis of adverse events for patients or individuals, whereby these adverse events belong to at least one of the following categories: undesired drug interactions, cancer diseases, CNS malfunctions, damage or disease, symptoms of aggression or behavioral disturbances; clinical, psychological and social consequences of brain damage, psychotic disturbances and personality disorders, dementia and/or associated syndromes, cardiovascular disease, malfunction and damage, malfunction, damage or disease of the gastrointestinal tract, malfunction, damage or disease of the respiratory system, lesion, inflammation, infection, immunity and/or convalescence, malfunction, damage or disease of the body as an abnormality in the development process, malfunction, damage or disease of the skin, of the muscles, of the connective tissue or of the bones, endocrine and metabolic malfunction, damage or disease, headaches or sexual malfunction This new method also serves in a particularly preferred manner for distinguishing cell types and tissues or for investigating cell differentiation.

Part of the present invention is also a kit, which consists oligonucleotides carrying a non-extendable 3' end, and a bisulfite reagent, wherein the non-extendable 3' terminus of the oligonucleotides is specific for a defined methylation status.

### Examples

### Example 1 : Selective amplification of unmethylated connexin 26 fragments with 3'amino modified oligonucleotides using polymerases with proofreading activity.

The LightCycler (Roche) is a device for conducting PCR and simultaneous detection and analysis of the PCR products. The device was modified according to the manufacturer's instructions using SybrGreen for the detection of dsDNA. The quantitative and qualitative analyses of the PCR were conducted with LightCycler Software Version 3.5. The connexin 26 fragment (accession number AF 144321.1 nt 748-905) was amplified from bisulfite treated methylated and unmethylated DNA.

The selective amplification of the unmethylated connexin 26 gene fragment was conducted in 20 µl of reaction volume (5 U of PfuI polymerase (Stratgen); 1x PfuI reaction buffer (Stratgene), 2mM MgCl2, 200 µM of each dNTP, 300 nM of each oligonucleotide, 3'modified by C3-amino residue (oligonucleotide F1C-3NH,GGTATATTGTTGAAAGTAATTGAATAAAATC-NH2, SEQ ID 1 ; oligonucleotide R1G-3NH, AAACAATACCCTCTAAAATAAAAATTAACG-NH2, SEQ ID 2), 0.25 g/µl BSA (Sigma, Munich), 0.25 µl of 1:1000 dilution of SybrGreen (Molecular Dynamics), 1ng of template DNA) with the following cycler program: 95°C - 10 min; 50 cycles: denaturation 96°C -10 s, annealing 56°C - 30 s; extension 72°C - 10 s. Detection was conducted at each amplification cycle by SybrGreen in the extension step after 10 s. The connexin 26-PCR fragment was detected monitoring the F1 channel (GGtAtATTGTTGAAAGTAATTGAATAAAATTGGAAATTTGAGAAGGTGTTTGTTTG GATTGGTGAGATTTTGAGGGGAGAAAGAAGTGGGGAtTTTGtTGGtAttAGTGGTGt ttttTttTTGGttAtTGTTAAtttttATTttAGAGGGtAtTGttt; Seq ID 3). Due to the identical base pair behavior of uracile and thymine the positions which correspond to the converted, originally unmethylated, cytosines are marked with a small "t" (resp. small "a" in the complementary strand). In contrast, capital "T" (resp. "A") describe thymines already existing prior to bisulfite treatment.

The amplification of the connexin fragment was investigated on 1ng methylated and unmethylated bisulfite treated DNA. Only with unmethylated DNA or mixtures of unmethylated and methylated bisulfite treated template DNA a amplification product was generated in the real time PCR. The melting point of the product was identical to that obtained with the unmodified primers (primerF2-T, GGTATATTGTTGAAAGTAATTGAATAAAATT; SEQ ID 4; primer R2-A AAACAATACCCTCTAAAATAAAAATTAACA, SEQ ID 5) with unmethylated bisulfite treated template DNA . The oligonucleotide F1C-3NH and R1-G-3NH will not be extended by polymerase without proofreading activity. PfuI shows 3'-5'exonuclease proofreading activity on primers that do not match at their 3'position with the template. The oligonucleotides F1C-3NH and R1-G-3NH show such mismatches with unmethylated bisulfite treated template and therefore the 3'-base, including the amino modification is removed. Subsequently, the remaining primer, shortened by one nucleotide acts as a primer for the PCR. Therefore, only the unmethylated connexin fragment is amplified.

### Example 2: Selective amplification of methylated connexin 26 fragments with 3' modified oligonucleotide using pyrophosphorolysis-activated exonuclease activity of polymerases.

The LightCycler (Roche) is a device for conducting PCR and simultaneous detection and analysis of the PCR products. The device was modified according to the manufacturer's instructions using SybrGreen for the detection of dsDNA. The quantitative and qualitative analyses of the PCR were conducted with LightCycler Software Version 3.5. The connexin 26 fragment (accession number AF 144321.1 , nt 748-905) was amplified from bisulfite treated methylated and unmethylated DNA.

The selective amplification of methylated connexin 26 gene fragments was conducted in 20 µl of reaction volume (5 U of FastStart Taq polymerase (Roche, Penzberg); 1x FastStart reaction buffer (Roche, Penzberg), 300 nM of each oligonucleotide, 3'modified by C3-amino residue (oligonucleotide F1C-ddC, GGTATATTGTTGAAAGTAATTGAATAAAATddC, SEQ ID 6; oligonucleotide R1G-A, AAACAATACCCTCTAAAATAAAAATTAACddg, SEQ ID 7), 0.25 µg/µl BSA (Sigma, Munich), 300 µM Na4P2O7 (Fluka), 0.25 µl of 1:1000 dilution of SybrGreen (Molecular Dynamics), 1ng of template DNA) with the following cycler program: 95°C - 10 min; 50 cycles: denaturation 96°C -10 s, annealing 56°C - 30 s; extension 72°C - 10 s. Detection was conducted at each amplification cycle by SybrGreen in the extension step after 10 s. The connexin 26-PCR fragment was detected monitoring the F1 channel (GGtAtATTGTTGAAAGTAATTGAATAAAATCGGAAATTCGAGAAGGCGTTCGTTCG GATTGGTGAGATTTTGAGGGGAGAAAGAAGCGGGGAtTTCGtCGGtAttAGCGGCGt ttttTttTCGGttAtCGTTAAtttttATTttAGAGGGtAtTGttt; SEQ ID 8). The amplification of the connexin fragment was investigated on 1ng methylated and unmethylated bisulfite treated DNA. Only with methylated DNA or mixtures of methylated and unmethylated bisulfite treated template DNA a exponential amplification of connexin was found in the real time PCR. The melting point of the product was identical to that obtained with the unmodified primers (primerF2-T, GGTATATTGTTGAAAGTAATTGAATAAAATC, SEQ ID 9; primer R2-A AAACAATACCCTCTAAAATAAAAATTAACG, SEQ ID 10) with methylated bisulfite treated template DNA.

The oligonucleotide F1C-ddC will not be extended by Fast-StartTaq polymerase without PPi in the PCR reaction. However, under PCR condition with 300 µM PPi the ddC-modified oligonucleotide gets activated by pyrophosphorolysis if the oligonucleotide matches at its 3'position with the template. The primer F1C-ddC matches to the methylated bisulfite treated template and therefore, the 3'-didesoxynucleotide is removed. Subsequently, the remaining oligonucleotide, shortened by one nucleotide, acts as a primer for the PCR. Therefore, only the methylated connexin fragment is exponentially amplified

## Claims

1. Method for the analysis of cytosine methylation in DNA, **characterized in that** the following steps are conducted:
a) a genomic DNA sample is chemically or enzymatically treated in such a way that all of the unmethylated cytosine bases are converted to uracil, while the 5-methylcytosine bases remain unchanged,
b) at least one oligonucleotide carrying a non-extendable 3' end is annealed to the converted DNA, wherein the non-extendable 3' terminus of the oligonucleotide is specific for a defined methylation status,
c) the non-extendable 3' terminus of the oligonucleotide is only removed in case the oligonucleotide is bound without mismatch to the DNA with the methylation status to be detected, while hybridization to the background DNA takes only place under mismatch formation,
d) the unblocked oligonucleotide is extended, and
e) the methylation status is concluded from the absence or presence of an extended oligonucleotide product.

2. A method according to one of the preceding claims, further **characterized in that** the 3' non-extendable terminus of the oligonucleotide is a nucleotide or nucleotide analogue which cannot be extended by a nucleic acid polymerase, but which can be removed by pyrophosphorolysis.

3. A method according to claim 2, further **characterized in that** the nucleotide or nucleotide analogue is selected from the group consisting of a 3'deoxynucleotide, a 2',3'-dideoxynucleotide, an acyclonucleotide, 3'-deoxyadenosine (cordycepin), 3'-azido-3'-deoxythymidine (AZT), 2',3'-dideoxyinosine (ddl), 2',3'-dideoxy-3'-thiacytidine (3TC) and 2',3'-didehydro-2',3'-dide- oxythymidine (d4T).

4. A method according to one of the preceding claims, further **characterized in that** the removal in step c) is performed with a DNA polymerase.

5. A method according to one of the preceding claims, further **characterized in that** the extension in step d) is performed with a DNA polymerase.

6. A method according to one of the preceding claims, further **characterized in that** in step e) the presence of an extended oligonucleotide product is detected by a label introduced by a nucleotide or nucleotide analog present in the extension step.

7. A method according to one of the preceding claims, further **characterized in that** in step e) the presence of an extended oligonucleotide product is detected by the binding or incorporation of a dye or spectral material.

8. A method according to claim 7, further **characterized in that** in step e) the presence of an extended oligonucleotide product is detected by real time probes.

9. A method according to one of the preceding claims, further **characterized in that** the steps b) to d) are performed bidirectioally.

10. A method according to one of the preceding claims, further **characterized in that** a exponential amplification is performed by repeating the steps b) to d) for several times.

11. A kit, which consists of oligonucleotides carrying a non-extendable 3' end, and a bisulfite reagent, wherein the non-extendable 3' terminus of the oligonucleotides is specific for a defined methylation status.

## Patentansprüche

1. Verfahren zur Analyse der Cytosinmethylierung in DNA, welches **dadurch gekennzeichnet ist, dass** die nachfolgenden Schritte durchgeführt werden:
a) eine genomische DNA-Probe wird chemisch oder enzymatisch derart behandelt, dass alle nicht-methylierten Cytosinbasen in Uracil umgewandelt werden, während die 5-Methylcytosinbasen unverändert bleiben,
b) mindestens ein Oligonukleotid, das ein nicht-verlängerbares 3'-Ende trägt, wird an die veränderte DNA hybridisiert, wobei der nicht-verlängerbare 3'-Terminus des Oligonukleotids spezifisch für einen bestimmten Methylierungsstatus ist,
c) der nicht verlängerbare 3'-Terminus des Oligonukleotids wird nur dann abgespalten, wenn das Oligonukleotid ohne Basenfehlpaarung (Mismatch) an die DNA gebunden ist, wobei der Methylierungsstatus nachweisbar ist, während die Hybridisierung an die Hintergrund-DNA nur unter Bildung von Basenfehlpaarungen erfolgt,
d) das nicht-blockierte Oligonukleotid wird verlängert, und
e) der Methylierungsstatus wird anhand des Vorhandenseins oder Fehlens eines verlängerten Oligonukleotidprodukts bestimmt.

2. Verfahren nach einem der vorhergehenden Ansprüche, welches ferner **dadurch gekennzeichnet ist, dass** der nicht verlängerbare 3'-Terminus des Oligonukleotids ein Nukleotid oder ein Nukleotidanalog ist, welches durch eine Nukleinsäurepolymerase nicht verlängert, jedoch mittels Pyrophosphorolyse abgespalten werden kann.

3. Verfahren nach Anspruch 2, welches ferner **dadurch gekennzeichnet ist, dass** das Nukleotid oder das Nukleotidanalog aus der Gruppe ausgewählt ist, bestehend aus einem 3'-Deoxynukleotid, einem 2',3'-Dideoxynukleotid, einem Acyclonukleotid, 3'-Deoxyadenosin (Cordycepin), 3'-Azido-3'-Deoxythymidin (AZT), 2',3'-Dideoxyinosin (ddl), 2',3'-Dideoxy-3'-thiacytidin (3TC) und 2',3'-Didehydro-2',3'-dideoxythymidin (d4T).

4. Verfahren nach einem der vorhergehenden Ansprüche, welches ferner **dadurch gekennzeichnet ist, dass** die Abspaltung in Schritt c) mit einer DNA-Polymerase durchgeführt wird.

5. Verfahren nach einem der vorhergehenden Ansprüche, welches ferner **dadurch gekennzeichnet ist, dass** die Verlängerung in Schritt d) mit einer DNA-Polymerase durchgeführt wird.

6. Verfahren nach einem der vorhergehenden Ansprüche, welches ferner **dadurch gekennzeichnet ist, dass** in Schritt e) das Vorhandensein eines verlängerten Oligonukleotidprodukts mit Hilfe eines Markers nachgewiesen wird, welcher durch ein Nukleotid oder ein Nukleotidanalog eingeschleust wird, das während des Schritts der Verlängerung vorliegt.

7. Verfahren nach einem der vorhergehenden Ansprüche, welches ferner **dadurch gekennzeichnet ist, dass** in Schritt e) das Vorhandensein eines verlängerten Oligonukleotidprodukts mit Hilfe der Bindung oder des Einschlusses eines Farbstoffes oder eines Spektralmaterials nachgewiesen wird.

8. Verfahren nach Anspruch 7, welches ferner **dadurch gekennzeichnet ist, dass** in Schritt e) das Vorhandensein eines verlängerten Oligonukleotidprodukts mit Hilfe von Echtzeit-Sonden nachgewiesen wird.

9. Verfahren nach einem der vorhergehenden Ansprüche, welches ferner **dadurch gekennzeichnet ist, dass** die Schritte b) bis d) bidirektional durchgeführt werden.

10. Verfahren nach einem der vorhergehenden Ansprüche, welches ferner **dadurch gekennzeichnet ist, dass** eine exponentielle Amplifikation durchgeführt wird, indem die Schritte b) bis d) mehrere Male wiederholt werden.

11. Ein Kit, bestehend aus Oligonukleotiden, die ein nicht verlängerbares 3'-Ende tragen, wobei der nicht verlängerbare 3'-Terminus der Oligonukleotide spezifisch für einen bestimmten Methylierungsstatus ist, sowie einem Bisulfit-Reagenz.

## Revendications

1. Méthode d'analyse de la méthylation de cytosine dans l'ADN, **caractérisée en ce que** les étapes suivantes sont réalisées :
a) un échantillon d'ADN génomique est traité chimiquement ou enzymatiquement de manière à ce que toutes les bases de cytosine non méthylées soient converties en uracil, les bases de 5-méthylcytosine restant inchangées ;
b) au moins un oligonucléotide portant une extrémité 3' non-extensible est annelé à l'ADN converti, étant donné que le terminal 3' non-extensible de l'oligonucléotide est spécifique pour un état de méthylation défini ;
c) le terminal 3' non-extensible de l'oligonucléotide est enlevé uniquement dans le cas où l'oligonucléotide est lié sans mésappariement à l'ADN à l'état à détecter, tandis que l'hybridation de l'ADN de fond a lieu seulement en cas de formation de mésappariement ;
d) l'oligonucléotide non bloqué est étendu, et
e) l'état de méthylation est conclu de l'absence ou de la présence d'un produit oligonucléotide étendu.

2. Méthode selon l'une quelconque des revendications précédentes, en outre **caractérisée en ce que** le terminal 3' non-extensible de l'oligonucléotide est un nucléotide ou analogue de nucléotide qui ne peut pas être étendu par une polymérase d'acide nucléique, mais qui peut être enlevé par pyrophosphorolyse.

3. Méthode selon la revendication 2, en outré **caractérisée en ce que** le nucléotide ou l'analogue de nucléotide est sélectionné dans le groupe constitué d'un 3'-deoxynucléotide, un 2',3'-dideoxynucléotide, un acyclonucléotide, 3'-deoxyadénosine (cordycépine), 3'-azido-3'-deoxythymidine (AZT), 2',3'-dideoxyinosine (ddl), 2',3'-dideoxy-3'-thiacytidine (3TC) et 2',3'-didehydro-2',3'-dideoxythymidine (d4T).

4. Méthode selon l'une quelconque des revendications précédentes, en outre **caractérisée en ce que** l'enlèvement à l'étape c) est effectué au moyen d'une polymérase d'ADN.

5. Méthode selon l'une quelconque des revendications précédentes, en outre **caractérisée en ce que** l'extension à l'étape d) est réalisée au moyens d'une polymérase d'ADN.

6. Méthode selon l'une quelconque des revendications précédentes, en outre **caractérisée en ce que**, à l'étape e), la présence d'un produit oligonucléotide étendu est détectée par une étiquette introduite par un nucléotide ou analogue de nucléotide présent à l'étape d'extension.

7. Méthode selon l'une quelconque des revendications précédentes, en outre **caractérisée en ce que**, à l'étape e), la présence d'un produit oligonucléotide étendu est détectée par la liaison ou l'incorporation d'un colorant ou d'un matériau spectral.

8. Méthode selon la revendication 7, en outre **caractérisée en ce que**, à l'étape e), la présence d'un produit oligonucléotide étendu est détectée par des sondes en temps réel.

9. Méthode selon l'une quelconque des revendications précédentes, en outre **caractérisée en ce que** les étapes b) à d) sont réalisées bidirectionnellement.

10. Méthode selon l'une quelconque des revendications précédentes, en outre **caractérisée en ce qu'**une amplification exponentielle est réalisée en répétant les étapes b) à d) plusieurs fois.

11. Kit consistant en oligonucléotides portant une extrémité 3' non-extensible et un réactif bisulfite, étant donné que le terminal 3' non-extensible des oligonucléotides est spécifique pour un état de méthylation défini.


### Sequence listing

<110> Epigenomics AG
<120> Method for the analysis of methylated DNA
<160> 10
<210> 1
   <211> 31
   <212> DNA
   <213> Artificial Sequence
<220>
   <223> chemically treated genomic DNA (Homo sapiens)
<400> 1
   ggtatattgt tgaaagtaat tgaataaaat c 31
<210> 2
   <211> 30
   <212> DNA
   <213> Artificial Sequence
<220>
   <223> chemically treated genomic DNA (Homo sapiens)
<400> 2
   aaacaatacc ctctaaaata aaaattaacg 30
<210> 3
   <211> 158
   <212> DNA
   <213> Artificial Sequence
<220>
   <223> chemically treated genomic DNA (Homo sapiens)
<400> 3
   ggtatattgt tgaaagtaat tgaataaaat tggaaatttg agaaggtgtt tgtttggatt      60
   ggtgagattt tgaggggaga aagaagtggg gattttgttg gtattagtgg tgtttttttt     120
   ttggttattg ttaattttta ttttagaggg tattgttt                              158
<210> 4
   <211> 31
   <212> DNA
   <213> Artificial Sequence
<220>
   <223> chemically treated genomic DNA (Homo sapiens)
<400> 4
   ggtatattgt tgaaagtaat tgaataaaat t 31
<210> 5
   <211> 31
   <212> DNA
   <213> Artificial Sequence
<220>
   <223> chemically treated genomic DNA (Homo sapiens)
<400> 5
   aaaacaatac cctctaaaat aaaaattaac a 31
<210> 6
   <211> 30
   <212> DNA
   <213> Artificial Sequence
<220>
   <223> chemically treated genomic DNA (Homo sapiens)
<400> 6
   ggtatattgt tgaaagtaat tgaataaaat 30
<210> 7
   <211> 29
   <212> DNA
   <213> Artificial Sequence
<220>
   <223> chemically treated genomic DNA (Homo sapiens)
<400> 7
   aaacaatacc ctctaaaata aaaattaac 29
<210> 8
   <211> 158
   <212> DNA
   <213> Artificial Sequence
<220>
   <223> chemically treated genomic DNA (Homo sapiens)
<400> 8
   ggtatattgt tgaaagtaat tgaataaaat cggaaattcg agaaggcgtt cgttcggatt      60
   ggtgagattt tgaggggaga aagaagcggg gatttcgtcg gtattagcgg cgtttttttt     120
   tcggttatcg ttaattttta ttttagaggg tattgttt                             158
<210> 9
   <211> 31
   <212> DNA
   <213> Artificial Sequence
<220>
   <223> chemically treated genomic DNA (Homo sapiens)
<400> 9
   ggtatattgt tgaaagtaat tgaataaaat t 31
<210> 10
   <211> 30
   <212> DNA
   <213> Artificial Sequence
<220>
   <223> chemically treated genomic DNA (Homo sapiens)
<400> 10
   aaacaatacc ctctaaaata aaaattaaca 30